(19) Europäisches Patentamt
European Patent Office
Office européen des brevets

(11) Numéro de publication : **0 512 890 A1**

(12) # DEMANDE DE BREVET EUROPEEN

(21) Numéro de dépôt : **92401192.7**

(51) Int. Cl.$^5$ : **C07D 215/46,** A61K 31/47

(22) Date de dépôt : **24.04.92**

(30) Priorité : **07.05.91 FR 9105571**

(43) Date de publication de la demande : **11.11.92 Bulletin 92/46**

(84) Etats contractants désignés : **AT CH DE FR GB IT LI NL**

(71) Demandeur : **RHONE-POULENC CHIMIE
25, quai Paul Doumer
F-92408 Courbevoie Cédex (FR)**

(72) Inventeur : **Chazal, Bruno M.
46, rue du Dr Ollier
F-69100 Villeurbanne (FR)**
Inventeur : **Mencarelli, Caroline
49, chemin de Beckensteiner
F-69260 Charbonniere (FR)**
Inventeur : **Roustan, Alain M.
75, chemin du Clos Burtin
F-69230 Saint Genis Laval (FR)**

(74) Mandataire : **Dutruc-Rosset, Marie-Claude et al
RHONE-POULENC CHIMIE Direction des Brevets 25, Quai Paul Doumer
F-92408 Courbevoie Cédex (FR)**

(54) **Procédé de préparation de sulfate de chloroquine.**

(57) La présente invention a pour objet un nouveau procédé de préparation de sulfate de chloroquine.

Le procédé de préparation de sulfate de chloroquine de l'invention est caractérisé par le fait qu'il consiste :

(1) - à précipiter le sulfate de chloroquine par traitement de la base chloroquine par l'acide sulfurique, en solution alcoolique,

(2) - à séparer le sulfate de chloroquine obtenu et éventuellement à le soumettre à un ou plusieurs lavages,

(3) - à le cristalliser dans une solution hydro-alcoolique par addition d'une cétone, de préférence l'acétone,

(4) - à séparer le sulfate de chloroquine purifié,

(5) - à lui faire subir éventuellement une opération de séchage.

Il permet d'accéder à un sulfate de chloroquine présentant une excellente pureté.

EP 0 512 890 A1

La présente invention a pour objet un nouveau procédé de préparation de sulfate de chloroquine.

La chloroquine est un produit connu doué de propriétés anti-malariques remarquables.

Le terme "chloroquine" désigne plus précisément la chloro-7 (diéthylamino-4 méthyl-1 butylamino)-4 quinoléïne.

Une de ses voies d'accès consiste à condenser la chloro-7 tétrahydro-1,2,3,4 quinolinone-4 avec la diéthylamino-4 méthyl-1 butylamine, en présence d'air selon le procédé décrit par W.S JOHNSON et B.G. BUELL, J. Amer. Chem. Soc. 74, p. 4513 (1952).

Un autre mode de préparation réside dans la condensation de la dichloro-4,7, quinoléïne avec la diéthylamino-4 méthyl-1 butylamine. Une telle réaction est décrite notamment par Charles C. PRICE et al dans J. Am. Chem. Soc., 68, p. 1214 et suivantes (1946).

Dans l'exposé qui suit de la présente invention, on désigne par "base chloroquine" le produit résultant de ladite condensation.

La chloroquine est disponible sur la marché, sous la forme d'un sel préparé à partir de la base chloroquine.

Elle se trouve soit sous la forme de diphosphate, soit sous la forme de sulfate.

Il existe dans l'état de la technique de nombreuses références décrivant le diphosphate de chloroquine. on peut se référer entre autres à l'article de Charles C. PRICE précité.

En ce qui concerne la voie sulfate, il y a peu de littérature à ce sujet car la préparation du sulfate de chloroquine directement à partir de la base chloroquine pose un problème car le produit résultant du traitement de la base chloroquine par l'acide sulfurique conduit à un produit très coloré, impropre pour le marché.

Pour pallier cet inconvénient, on a proposé de préparer le sulfate de chloroquine, en faisant réagir la base chloroquine en solution dans le benzène avec de l'acide sulfurique (RO 74-77924), puis d'effectuer la purification du sulfate de chloroquine obtenu, en effectuant l'extraction de la base chloroquine dans une phase organique (benzène, toluène et chloroforme) et le traitement de la base par l'acide sulfurique à pH 4,5 - 5,0 (RO 74-83889).

Un tel procédé ne peut en aucun cas être satisfaisant car il fait appel au benzène, solvant industriellement prohibé en raison de sa toxicité et de son caractère cancérigène.

L'objet de la présente invention est de fournir un procédé qui permet d'accéder directement au sulfate de chloroquine à partir de la base chloroquine et qui permet d'obvier aux inconvénients précités.

Plus précisément, l'invention a pour objet un procédé de préparation de sulfate de chloroquine caractérisé par le fait qu'il consiste :

(1) - à précipiter le sulfate de chloroquine par traitement de la base chloroquine par l'acide sulfurique, en solution alcoolique,

(2) - à séparer le sulfate de chloroquine obtenu et éventuellement à le soumettre à un ou plusieurs lavages,

(3) - à le cristalliser dans une solution alcoolique par addition d'une cétone, de préférence l'acétone,

(4) - à séparer le sulfate de chloroquine purifié,

(5) - à lui faire subir éventuellement une opération de séchage.

Selon un mode préféré de réalisation de l'invention, on effectue deux cristallisations successives du sulfate de chloroquine.

Conformément au procédé de l'invention, on effectue dans une première étape, la précipitation du sulfate de chloroquine impur à partir de la base de chloroquine.

La base chloroquine comme mentionné précédemment, résulte de la réaction entre la dichloro-4,7 quinoléïne et de la diéthylamino-4 méthyl-1 butylamine.

La dichloro-4,7 quinoléïne est un produit connu décrit notamment par Charles C. PRICE et al dans J. Am. Chem. Soc., 68, p.1204 et suivantes (1946).

Elle peut être obtenue, en autres, par réaction entre la chloro-3 aniline et l'éthoxyméthylènemalonate d'éthyle, cyclisation thermique à une température de 250°C du produit obtenu permettant ainsi d'obtenir la chloro-7 hydroxy-4 carbéthoxy-3 quinoléïne, hydrolyse, décarboxylation vers 250°C - 270°C, traitement de l'hydroxy-4 chloro-7 quinoléïne obtenue par l'oxychlorure de phosphore conduisant ainsi à la dichloro-4,7 quinoléïne.

La base chloroquine qui intervient comme produit de départ dans le procédé de l'invention, résulte donc de la réaction de condensation bien connue de l'Homme de métier, de la dichloro-4,7 quinoléïne avec la diéthylamino-4 méthyl-1 butylamine.

Dans le procédé de d'invention, on part d'une base chloroquine préparée, de préférence, à partir d'une dichloro-4,7 quinoléïne présentant une teneur en dichloro-4,5 quinoléïne inférieure à 10 % et d'une diéthylamino-4 méthyl-1 butylamine ayant une pureté supérieure à 97 % et contenant moins de 10 % d'eau.

La première étape du procédé de l'invention consiste à diluer la base chloroquine dans une solution alcoolique, de préférence une solution éthanolique.

La teneur de la base chloroquine dans la solution alcoolique peut varier très largement entre 150 et 250

g/l, mais, est choisie avantageusement entre 200 et 250 g/l.

On refroidit ensuite la solution éthanolique de base chloroquine à une température qui se situe, de préférence, entre 15°C et 20°C.

On effectue ensuite la précipitation du sulfate de chloroquine par addition d'acide sulfurique dans la solution éthanolique de base chloroquine.

On fait appel, de préférence, à un acide sulfurique concentré généralement à 95 %.

La quantité d'acide sulfurique additionnée est de préférence au moins égale à la stoechiométrie de la réaction (I) définie ci-après mais elle peut être également en excès par rapport à la stoechiométrie :

ledit excès pouvant atteindre par exemple, jusqu'à 20 % de la quantité stoechiomètrique.

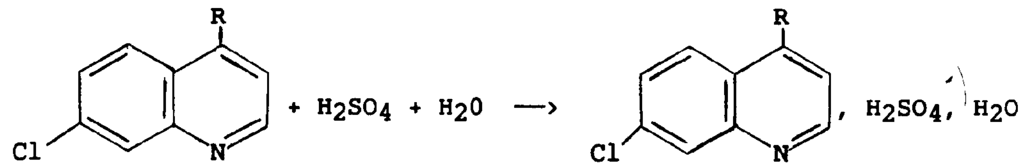


(I)

dans lesdites formules,

R représente le radical

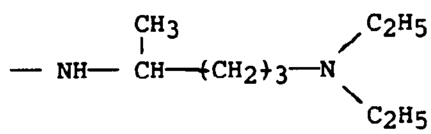

Un moyen aisé d'ajuster la quantité d'acide sulfurique à mettre en oeuvre et de contrôler le pH qui est, de préférence, supérieur ou égal à 5 et inférieur ou égal à 6.

Il est à noter que la borne supérieure ne présente aucun caractère critique mais le choix d'un pH supérieur à 6 conduit à une perte de rendement, du produit de la réaction.

On additionne progressivement, de préférence, l'acide sulfurique dans la solution éthanolique de la base chloroquine.

Tout au cours de l'addition de l'acide sulfurique, il est souhaitable que la température du milieu réactionnel ne dépasse pas environ 40°C.

Ainsi, il peut être nécessaire de refroidir le milieu réactionnel afin de satisfaire cette exigence.

En fin d'addition de l'acide sulfurique, on obtient une suspension de sulfate de chloroquine qui est refroidie à une température qui se situe, de préférence, entre 15°C et 25°C.

Afin de parfaire la précipitation, on maintient avantageusement sous agitation et à cette température, la suspension obtenue pendant une durée variable allant de 30 minutes à deux heures et, de préférence, égale à environ 1 heure.

On sépare le sulfate de chloroquine précipité selon les techniques classiques de séparation solide/liquide telles que par exemple, filtration, centrifugation, décantation ou essorage.

D'une manière préférée, on sépare le précipité par filtration.

Après élimination des eaux-mères, le précipité récupéré est soumis à un ou plusieurs lavages effectués à l'aide d'un solvant organique, de préférence, l'éthanol.

On conduit l'opération de lavage, soit par addition directe du solvant de lavage sur le gâteau de filtration, soit par remise en suspension du précipité dans le solvant de lavage.

Le nombre de lavages est déterminé de telle sorte que le liquide surnageant soit clair, c'est-à-dire non coloré. Généralement, trois lavages suffisent.

Le précipité est à nouveau séparé selon les techniques précitées puis ensuite mis en solution.

Selon le procédé de l'invention, on réalise dans une seconde étape, la cristallisation du sulfate de chloroquine.

A cet effet, on charge de l'eau, de préférence distillée en une quantité représentant de 5 à 7 fois, le volume d'acide sulfurique précédemment chargé. La quantité préférée représente environ 6 fois le volume d'acide sulfurique chargé.

On agite et l'on chauffe le milieu réactionnel à sa température de reflux, généralement voisine de 85°C pendant une durée suffisante permettant d'obtenir la dissolution complète du précipité.

En fin de chauffage, il y a lieu de veiller à ce que la densité de la solution soit comprise dans un intervalle allant de 1,060 à 1,080 de préférence, comprise entre 1,070 à 1,080. Les densités données correspondent à des densités mesurées à 30°C.

Si nécessaire, la densité est réajustée de telle sorte qu'elle soit dans l'intervalle précité.
On additionne de l'éthanol si la densité est trop élevée. On concentre la solution par distillation si la densité se trouve en dessous de la limite inférieure.

On conduit ensuite la cristallisation du sulfate de chloroquine. Pour ce faire, on refroidit la solution obtenue précédemment à une température inférieure à 15°C, de préférence, voisine 11°C - 12°C.
Cette température peut être obtenue, entre autres, par refroidissement à l'aide d'un mélange réfrigérant eau/chlorure de sodium appelé couramment "saumure".

Lorsque la solution est refroidie, on ajoute rapidement, environ en moins de 15 minutes, de l'acétone.

On charge de l'acétone en une quantité représentant de 2 à 4 fois, le volume d'eau précédemment chargée. La quantité préférée représente environ 3 fois le volume d'eau chargée.

Lors de l'addition de l'acétone, on note une élévation de température inférieure à 10°C, généralement, de 5°C - 6°C.

On ramène la suspension à la température de refroidissement prédéfinie et lorsque la température précitée est atteinte, on maintient encore celle-ci pendant un certain temps, généralement une dizaine de minutes.

On sépare le précipité selon les techniques classiques de séparation, de préférence par filtration. Les eaux-mères sont éliminées.

On effectue de préférence un lavage du précipité séparé à l'aide d'un mélange de solvants organiques.

On fait appel, de préférence, à un mélange d'acétone et d'éthanol comprenant, par exemple, de 60 à 70 % d'acétone et de 30 à 40 % d'éthanol.

On peut répéter cette opération de lavage.

Le ou les lavages à l'aide du mélange acétone/alcool sont poursuivis jusqu'à ce que les eaux-mères récupérées soient incolores.

On termine l'opération de lavage, par un lavage à l'acétone uniquement.

Selon une variante préférée du procédé de l'invention, on effectue une seconde cristallisation afin d'obtenir un sulfate de chloroquine pur.

Le précipité séparé et lavé est ensuite remis en solution.

A cet effet, on charge de l'eau, de préférence distillée en une quantité représentant de 3,5 à 5 fois, le volume d'acide sulfurique précédemment chargé. La quantité préférée représente environ 4 fois le volume d'acide sulfurique chargé.

On agite et l'on chauffe à la température de reflux du mélange réactionnel, généralement, voisine de 85°C pendant une durée suffisante permettant d'obtenir la dissolution complète du précipité.

On veille à ce qu'il y ait une complète dissolution du précipité.

En fin de chauffage, on ajoute une certaine quantité d'eau, de préférence distillée qui correspond à la quantité d'eau qui a été éliminée au cours de la distillation précédente.

Puis, on additionne une quantité d'éthanol qui correspond à la quantité d'eau qui a été ajoutée, juste après la séparation du précipité afin de le remettre en solution.

Ensuite, on refroidit la solution obtenue précédemment à une température inférieure à 25°C, de préférence, voisine 20°C,

On conduit ensuite la cristallisation du sulfate de chloroquine.

Lorsque la solution est à la température souhaitée, on ajoute rapidement, environ en moins de 15 minutes, de l'acétone.

On charge de l'acétone en une quantité représentant de 2 à 4 fois, le volume d'eau précédemment chargée. La quantité préférée représente environ 3 fois le volume d'eau chargée.

On chauffe la suspension obtenue à une température allant de 30°C à 50 °C, de préférence, voisine de 40°C.

Enfin, on refroidit la suspension à la température comprise entre 15°C et 25°C, de préférence, choisie aux environs de 20°C.

On sépare le précipité selon les techniques classiques de séparation, de préférence par centrifugation.

Les eaux-mères sont éliminées.

On peut répéter l'opération de 1 à 4 fois.

A chaque opération de centrifugation, on effectue un lavage du précipité à l'acétone.

Après récupération du précipité de sulfate de chloroquine, on le soumet à une opération classique de séchage. On choisit, de préférence, une température de séchage comprise entre 80°C et 100°C.

Afin de réduire la durée de l'opération, celui-ci peut être conduit sous pression réduite, par exemple, inférieure à 100 mm de mercure (133,3 . $10^2$ Pa) , mais, de préférence, comprise entre 5 et 80 mm de mercure (667 Pa et 107 . $10^2$ Pa).

On obtient conformément au procédé de l'invention, un sulfate de chloroquine blanc, d'une excellente pureté supérieure à 99 %. Il présente une teneur en eau de 4 à 5 % et une teneur en cendres au maximum égale à 0,1 %.

On donne ci-après un exemple de mise en oeuvre du procédé de l'invention.

Cet exemple est donné à titre illustratif, sans caractère limitatif.

Exemple 1

On part d'une base chloroquine qui résulte de la réaction entre la dichloro-4,7 quinoléïne (contenant moins de 10 % d'isomère de dichloro-4,5 quinoléïne) et la diéthylamino-4 méthyl-1 butylamine.

On commence par effectuer une dilution de la base chloroquine.

Dans un réacteur à double enveloppe de 2 litres équipé d'une agitation centrale à hélice et à contre-pale, on charge :

- 870 $cm^3$ d'éthanol à 95 %,
- 184 g de base chloroquine.

On refroidit ensuite la solution éthanolique de base chloroquine à une température de 20°C.

On effectue ensuite la précipitation du sulfate de chloroquine par addition progressive d'acide sulfurique dans la solution éthanolique de base chloroquine.

On fait appel à un acide sulfurique commercial, concentré à 95 % (d = 1, 834).

La quantité d'acide sulfurique additionnée est telle que le pH obtenu soit de 5.

Tout au cours de l'addition de l'acide sulfurique, on refroidit le milieu réactionnel afin que sa température ne dépasse pas 40°C.

En fin d'addition de l'acide sulfurique, on obtient une suspension de sulfate de chloroquine qui est refroidie à une température qui se situe vers 20°C.

Afin de parfaire la précipitation, on maintient avantageusement sous agitation et à cette température, la suspension obtenue pendant une durée de 1 heure.

On sépare le sulfate de chloroquine précipité, par filtration.

Après élimination des eaux-mères, on lave le gâteau de filtration, à l'aide de 3 fois 80 $cm^3$ d'éthanol et l'on répète, si nécessaire, l'opération jusqu'à ce que le filtrat soit décoloré.

On sépare le précipité, par filtration.

On charge 210 $cm^3$ d'eau distillée.

On agite et l'on chauffe le milieu réactionnel à sa température de reflux de 80°C, pendant une heure.

On veille à ce qu'il y ait une complète dissolution du précipité.

En fin de chauffage, on additionne de l'éthanol de telle sorte que la densité de la solution soit de 1,075.

On conduit ensuite la cristallisation du sulfate de chloroquine. Pour ce faire, on refroidit à l'aide de saumure, la solution obtenue précédemment à une température de 11°C - 12°C.

Lorsque la solution est refroidie, on ajoute en 15 minutes, 630 $cm^3$ d'acétone.

En fin d'addition d'acétone, on ramène la suspension à la température de 13°C, et l'on maintient la suspension à cette température, pendant 15 minutes .

On sépare le précipité par filtration. Les eaux-mères sont éliminées.

On effectue un premier lavage du précipité séparé à l'aide de 280 $cm^3$ d'un mélange de 70 % d'acétone et de 30 % d'éthanol puis 2 lavages avec 40 $cm^3$ de ce mélange.

On termine par un lavage avec 40 $cm^3$ d'acétone.

On effectue une seconde cristallisation afin d'obtenir un sulfate de chloroquine pur.

Le précipité séparé et lavé est ensuite remis en solution. A cet effet, on charge 147 $cm^3$ d'eau distillée.

On agite et l'on chauffe à la température de reflux du mélange réactionnel à 85°C pendant une heure.

On veille à ce qu'il y ait une complète dissolution du précipité.

En fin de chauffage, on ajoute 2 $cm^3$ d'eau distillée.

Puis, on additionne 147 $cm^3$ d'éthanol.

On refroidit la solution obtenue précédemment à une température de 20°C.

On conduit ensuite la cristallisation du sulfate de chloroquine.

Lorsque la solution est à 20°C, on ajoute environ en 15 minutes, 440 $cm^3$ d'acétone.

On chauffe la suspension obtenue à une température de 40°C.

Enfin, on refroidit la suspension à la température de 20°C.

On sépare le précipité par filtration. Les eaux-mères sont éliminées.

On soumet le gâteau de filtration à des lavages à l'acétone, à raison de 3 fois 20 $cm^3$.

Après récupération du précipité de sulfate de chloroquine, on le sèche à l'étuve à une température de 90°C.

On obtient un sulfate de chloroquine ayant une pureté d'au moins de 99 % et présentant les caractéristiques suivantes :

- eau : 4,39 %,
- cendres sulfurique après calcination : 0,07 %,
- chlorure : < 0,0005 %,
- métaux lourds (Pb) : < 0,002 %.

## Revendications

**1.** Procédé de préparation de sulfate de chloroquine caractérisé par le fait qu'il consiste :
(1) - à précipiter le sulfate de chloroquine par traitement de la base chloroquine par l'acide sulfurique, en solution alcoolique,
(2) - à séparer le sulfate de chloroquine obtenu et éventuellement à le soumettre à un ou plusieurs lavages,
(3) - à le cristalliser dans une solution alcoolique par addition d'une cétone, de préférence l'acétone,
(4) - à séparer le sulfate de chloroquine purifié,
(5) - à lui faire subir éventuellement une opération de séchage.

**2.** Procédé selon la revendication 1 caractérisé par le fait que l'on effectue deux cristallisations successives du sulfate de chloroquine.

**3.** Procédé selon l'une des revendications 1 et 2 caractérisé par le fait que l'on effectue dans une première étape, la précipitation du sulfate de chloroquine impur à partir de la base de chloroquine selon les opérations suivantes :
- on dilue la base chloroquine dans une solution alcoolique, de préférence, une solution éthanolique,
- on refroidit ensuite la solution éthanolique de base chloroquine à une température qui se situe, de préférence, entre 15°C et 20°C,
- on additionne progressivement de l'acide sulfurique dans la solution éthanolique de base chloroquine en une quantité telle que l'on contrôle le pH de telle sorte qu'il soit supérieur ou égal à 5 et inférieur ou égal à 6,
- on sépare le sulfate de chloroquine précipité, de préférence, par filtration.

**4.** Procédé selon la revendication 3 caractérisé par le fait que la teneur de la base chloroquine dans la solution alcoolique varie entre 150 et 250 g/l et de préférence, entre 200 et 250 g/l.

**5.** Procédé selon l'une des revendications 3 et 4 caractérisé par le fait que lors de l'addition de l'acide sulfurique dans la solution éthanolique de base chloroquine, on maintient la température du milieu réactionnel à une température inférieure à environ 40°C, puis l'on refroidit la suspension de sulfate de chloroquine à une température qui se situe de préférence entre 15°C et 25°C et ensuite l'on maintient avantageusement sous agitation et à cette température, la suspension obtenue pendant une durée variable allant de 30 minutes à deux heures et, de préférence, égale à environ 1 heure.

**6.** Procédé selon l'une des revendications 3 à 5 caractérisé par le fait que l'on soumet le précipité récupéré à un ou plusieurs lavages, à l'aide d'un solvant organique, de préférence l'éthanol, soit par addition directe du solvant de lavage sur le gâteau de filtration, soit par remise en suspension du précipité dans le solvant de lavage, puis l'on sépare à nouveau le précipité, après lavage.

**7.** Procédé selon l'une des revendications 1 à 6 caractérisé par le fait que l'on effectue dans une deuxième étape, la cristallisation du sulfate de chloroquine selon les opérations suivantes :
- on met le sulfate de chloroquine en solution en chargeant de l'eau en une quantité représentant de 5 à 7 fois, de préférence 6 fois, le volume d'acide sulfurique précédemment chargé,
- on agite et l'on chauffe le milieu réactionnel à sa température de reflux , pendant une durée suffisante permettant d'obtenir la dissolution complète du précipité,
- on ajuste la densité de la solution de telle sorte qu'elle soit comprise dans un intervalle allant de 1,060 à 1,080 de préférence, comprise entre 1,070 à 1,080,
- on refroidit la solution obtenue précédemment à une température inférieure à 15°C, de préférence voisine 11°C - 12°C,
- on ajoute rapidement de l'acétone en une quantité représentant de 2 à 4 fois, de préférence 3 fois le volume d'eau précédemment chargée,
- on ramène la suspension à la température de refroidissement prédéfinie que l'on maintient encore pendant un certain temps,
- on sépare le précipité, de préférence par filtration.

**8.** Procédé selon la revendication 7 caractérisé par le fait que l'on effectue un ou plusieurs lavages du précipité séparé à l'aide d'un mélange d'acétone et d'éthanol comprenant de 60 à 70 % d'acétone et de 30 à 40 % d'éthanol et que l'on termine par un lavage à l'acétone.

**9.** Procédé selon l'une des revendications 1 à 8 caractérisé par le fait que l'on effectue une deuxième cristallisation du sulfate de chloroquine selon les opérations suivantes :
- on met le sulfate de chloroquine en solution en chargeant de l'eau en une quantité représentant de 3,5 à 5 fois, de préférence 4 fois, le volume d'acide sulfurique précédemment chargé,
- on agite et l'on chauffe le milieu réactionnel à sa température de reflux pendant une durée suffisante permettant d'obtenir la dissolution complète du précipité,
- on ajoute une certaine quantité d'eau qui correspond à la quantité d'eau qui a été éliminée au cours de la distillation précédente,
- on additionne une quantité d'éthanol qui correspond la quantité d'eau qui a été ajoutée, juste après la séparation du précipité afin de le remettre en solution,
- on refroidit la solution obtenue précédemment à une température inférieure à 25°C, de préférence voisine 20°C,
- on ajoute rapidement de l'acétone en une quantité représentant de 2 à 4 fois, de préférence 3 fois le volume d'eau précédemment chargée,
- on chauffe la suspension obtenue à une température allant de 30°C à 50 °C, de préférence voisine de 40°C,
- on refroidit la suspension à la température comprise entre 15°C et 25°C, de préférence choisie aux environs de 20°C,
- on sépare le précipité, de préférence par centrifugation.

**10.** Procédé selon la revendication 9 caractérisé par le fait que l'on répète l'opération de centrifugation 1 à 4 fois et que l'on effectue à chaque opération, un lavage du précipité à l'acétone.

**11.** Procédé selon l'une des revendications 1 à 10 caractérisé par le fait que l'on soumet le sulfate de chloroquine obtenu à une opération de séchage à une température de séchage comprise entre 80°C et 100°C.

**12.** Procédé selon la revendication 11 caractérisé par le fait que le séchage est conduit sous pression réduite inférieure à 100 mm de mercure mais, de préférence, comprise entre 5 et 80 mm de mercure.

Office européen des brevets

# RAPPORT DE RECHERCHE EUROPEENNE

Numero de la demande

EP 92 40 1192

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X,D | RO-A-61 257 (INSTITUTUL DE CERCETARI CHIMICO-FARMACEUTICE) 20 Avril 1976<br>* le document en entier *<br>--- | 1 | C07D215/46<br>A61K31/47 |
| X | RO-A-63 271 (INSTITUTUL DE CERCETARI CHIMICO-FARMACEUTICE) 20 Février 1978<br>* le document en entier *<br>----- | 1 | |
| | | | DOMAINES TECHNIQUES RECHERCHES (Int. Cl.5 ) |
| | | | C07D |

Le présent rapport a été établi pour toutes les revendications

| Lieu de la recherche | Date d'achèvement de la recherche | Examinateur |
|---|---|---|
| LA HAYE | 06 AOUT 1992 | DE JONG B.S. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)